(19) Europäisches Patentamt
European Patent Office
Office européen des brevets

(11) **EP 0 917 588 B2**

(12) **NOUVEAU FASCICULE DE BREVET EUROPEEN**
Après la procédure d'opposition

(45) Date de publication et mention de la décision concernant l'opposition:
**09.11.2011 Bulletin 2011/45**

(45) Mention de la délivrance du brevet:
**28.11.2001 Bulletin 2001/48**

(21) Numéro de dépôt: **97932670.9**

(22) Date de dépôt: **25.07.1997**

(51) Int Cl.:
**C12P 19/14** *(2006.01)*    **A23L 1/0528** *(2006.01)*

(86) Numéro de dépôt international:
**PCT/BE1997/000087**

(87) Numéro de publication internationale:
**WO 1998/005793 (12.02.1998 Gazette 1998/06)**

(54) **PROCEDE DE PREPARATION D'UNE COMPOSITION POLYDISPERSEE DE SACCHARIDES ET COMPOSITION POLYDISPERSEE DE SACCHARIDES OBTENUE**

VERFAHREN ZUR HERSTELLUNG EINER POLYDISPERSEN ZUSAMMENSETZUNG VON SACCHARIDEN UND DIE SO ERHALTENE POLYDISPERSE SACCHARIDE-ZUSAMMENSETZUNG

METHOD FOR PREPARING A POLYDISPERSED SACCHARIDE COMPOSITION AND RESULTING POLYDISPERSED SACCHARIDE COMPOSITION

(84) Etats contractants désignés:
**AT BE CH DE DK ES FI FR GB GR IE IT LI LU NL PT SE**
Etats d'extension désignés:
**AL LT LV RO SI**

(30) Priorité: **01.08.1996 BE 9600676**

(43) Date de publication de la demande:
**26.05.1999 Bulletin 1999/21**

(73) Titulaire: **RAFFINERIE TIRLEMONTOISE**
**1150 Bruxelles (BE)**

(72) Inventeurs:
• **DE LEENHEER, Leen**
  **B-3080 Tervuren (BE)**
• **BOOTEN, Karl**
  **B-3450 Geetbeets (BE)**

(74) Mandataire: **Koster, Nico**
**Tiense Suikerraffinaderij**
**Patent Department**
**Aandorenstraat 1**
**3300 Tienen (BE)**

(56) Documents cités:
**WO-A-93/00067     WO-A-97/23511**
**US-A- 4 871 574**

• **PATENT ABSTRACTS OF JAPAN vol. 12, no. 96 (C-484), 29 mars 1988 & JP 62 228293 A (MITSUI TOATSU CHEM INC), 7 octobre 1987, & DATABASE WPI Week 8704 Derwent Publications Ltd., London, GB; AN 323413**
• **PATENT ABSTRACTS OF JAPAN vol. 18, no. 219 (C-1192), 20 avril 1994 & JP 06 014792 A (MITSUI TOATSU CHEM INC), 25 janvier 1994, cité dans la demande & DATABASE WPI Week 9403 Derwent Publications Ltd., London, GB; AN 61482**
• **PRODUCT SHEET CONCERNANT LE PRODUIT RAFTILOSE®L95 (A1) Mai 1995,**
• **SCIENCE AND TECHNOLOGY OF FRUCTANS, CRC PRESS (A3) 1993, pages 172,187 - 276,288,300**
• **A. DE BRUYN ET AL. CARBOHYDRATE RESEARCH (A5) vol. 235, 1992, pages 303 - 308**
• **L. ZITTAN STARCH / STÄRKE (A6) vol. 33, 1981, pages 373 - 377**
• **M. VOGEL STUDIES IN PLANT SCIENCE, EDITED BY A. FUCHS (A7) 1993, pages 65 - 76**
• **LE SUCRE, LES SUCRES, LES ÉDULCORANTS ET LES GLUCIDES DE CHARGE DANS LES I.A.A. (A10) 1992, TEC & DOC, LAVOISIER, pages 276,277 - 304,305**
• **THE PROPERTIES OF RAFTILOSE®-RAFTILINE® 07 Novembre 1990,**

Remarques:
Le dossier contient des informations techniques présentées postérieurement au dépôt de la demande et ne figurant pas dans le présent fascicule.

EP 0 917 588 B2

**Description**

La présente invention se rapporte à un procédé de préparation d'une composition polydispersée de saccharides pauvre en glucose (G), fructose (F) et saccharose (GF), comprenant au moins 93,5% en poids par matière sèche (MS) de fructo-oligosaccharides constitués de chaînes d'unités de fructose de la formule $F_m$ et de chaînes d'unités de fructose avec un glucose terminal de la formule $GF_n$, n et m étant compris entre 2 et 20, de préférence entre 2 et 10, plus particulièrement entre 2 et 9, suivant lequel une matière contenant des fructanes est soumise à une hydrolyse partielle.

Un tel procédé est décrit dans le brevet EP-B-0440074, qui concerne un procédé de fabrication d'un produit inulo-oligosaccharide pauvre en glucose, fructose et saccharose. La matière contenant des fructanes est une matière végétale contenant de l'inuline qui est soumise, directement après broyage et pasteurisation, à un traitement enzymatique utilisant une endo-inulinase. On obtient ainsi une bouillie partiellement hydrolysée que l'on filtre, et on obtient un filtrat contenant 12,5% de disaccharides, dont 66% de saccharose et 34% d'inulobiose. Ensuite, un traitement du filtrat par une alpha-glucosidase a lieu pour en éliminer le saccharose, c'est-à-dire que le saccharose est transformé en glucose et fructose. Le glucose et le fructose sont ensuite enlevés par séparation chromatographique.

Ce procédé présente l'inconvénient d'exiger deux traitements enzymatiques et une séparation chromatographique, ce qui augmente son coût et sa complexité.

Un tel procédé est également décrit dans l'article "Isolation and Identification of O-β-D-fructofuranosyl - (2→1) - O-β-fructofuranosyl - (2→1)-D-fructose, a product of the enzymic hydrolysis of the inulin from Cichorium intybus" (A. De Bruyn et al., Carbohydrate Research, 235, pp. 303-308 (1992)). Dans le procédé décrit dans cet article, on utilise comme matière contenant les fructanes, de l'inuline extraite de racines de chicorée (Cichorium intybus). Cette inuline est soumise à un traitement enzymatique utilisant une endo-inulinase. On obtient ainsi un produit sirupeux brut contenant 85% en poids par matière sèche (MS) de fructo-oligosaccharides. Ce produit brut est ensuite enrichi par chromatographie en produisant une fraction enrichie contenant 95% de fructo-oligosaccharides (principalement des $GF_n$) et une fraction pauvre contenant 55% de fructo-oligosaccharides ($GF_n + F_m$).

Bien que ce procédé connu ne requière qu'un traitement enzymatique, une séparation chromatographique reste indispensable. De plus, la fraction contenant 55% de fructo-oligosaccharides est un sous-produit qui a peu de valeur du point de vue commercial.

Un autre procédé de production d'inulo-oligosaccharides pauvres en glucose, fructose et saccharose est décrit dans la demande de brevet japonais JP-5-21074 (publication JP-6-14792). Selon ce procédé, une solution aqueuse de 20% d'inuline, extraite de racines de chicorée, est soumise à un traitement enzymatique utilisant une endo-enzyme isolée d'une culture de Penicillum purpurogenum var. Rubriscerotium. Après une hydrolyse partielle, dont la limite de décomposition est environ 50%, les sucres réducteurs étant calculés comme fructose, la solution obtenue est purifiée par un traitement avec des résines échangeuses d'ions et de charbon actif, et ensuite, la solution est concentrée et le produit obtenu est séché. L'analyse par gel chromatographie du produit obtenu indique une teneur en :

DP1 : 1.5%; DP2 : 3.3%; DP3 : 31.4%; DP4 : 26.6%;
DP5 : 20.4%; DP6 : 13.3%; DP > 6 : 3.5%

Les résultats d'une hydrolyse partielle similaire effectuée sur de l'inuline par une endo-enzyme obtenue d'une culture de Penicillum trzebinskii indique une teneur en :

DP1 : 1.3%; DP2 : 0.9%; DP3 : 26.5%; DP4 : 27.6%;
DP5 : 18.5%; DP6 : 14.28%; DP > 6 : 11.0%

Cependant, dans cette analyse du produit fini par gel chromatographie, les résultats obtenus ne se rapportent essentiellement que sur la fraction d'inulo-oligosaccharides ayant un DP de maximum environ 8, et que les fructo-oligosaccharides ayant un DP plus élevé, bien que présents, ne sont pas quantifiables par cette méthode.

Le but de la présente invention est de fournir un procédé tel que défini ci-dessus, qui permet d'éviter les inconvénients des procédés de l'état de la technique connu.

La présente invention concerne en particulier un procédé de préparation d'une composition polydispersée de saccharides pauvre en glucose (G), fructose (F) et saccharose (GF) comprenant au moins 93,5% en poids par matière sèche (MS) de fructo-oligosaccharides constitués de chaînes d'unités de fructose de la formule Fm et de chaînes d'unités de fructose avec un glucose terminal de la formule $GF_n$, n et m étant compris entre 2 et 20, de préférence entre, 2 et 10, plus particulièrement entre 2 et 9, suivant lequel une matière contenant des fructanes est soumise à une hydrolyse partielle. A cet effet, selon l'invention, la matière contenant des fructanes comprend des fructanes de degré de polymérisation (DP) moyen d'au moins 7, c'est-à-dire supérieur ou égal à 7, et comprend tout au plus 3,5% en poids par matière sèche au total de glucose, de fructose et de saccharose.

Il est à noter que la composition polydispersée de saccharides obtenue par le procédé de préparation selon l'invention est essentiellement exempte d'inulo-oligosaccharides ayant un degré de polymérisation de plus de 10, de préférence plus de 9, et que, par conséquent, sa solution aqueuse à une concentration de 75%, et normalement même de 77%, en poids en matière sèche, reste limpide lors d'un stockage prolongé, même pendant plusieurs années, de préférence à température ambiante. Cette caractéristique apporte au produit obtenu par le procédé selon l'invention un avantage considérable sur le plan technico-commercial vis-à-vis des produits obtenus par un procédé connu de l'art.

En effet, il a été trouvé que, lorsque l'on utilise dans l'hydrolyse partielle ladite matière, on obtient directement une composition polydispersée comprenant au moins 93,5% par MS de fructo-oligosaccharides sans nécessiter une séparation chromatographique. Avantageusement, la composition polydispersée comprend au moins 95% de fructo-oligosaccharides et ladite matière comprend des fructanes contenant tout au plus 2% de F, G et GF au total.

De préférence, la matière contenant des fructanes comprend tout au plus 1% de F, G et GF au total.

Par "fructane", on entend tout composé dont un ou plusieurs liens fructosyl-fructose constituent la plupart des liens, tel qu'indiqué dans le document "Glossary of Fructan Terms" (A. L. Waterhouse et al., Science and Technology of Fructans, pp. 1-7 (1993)), incorporé ici par référence.

Par "degré de polymérisation (DP) moyen", on entend le degré de polymérisation moyen ($\overline{DP}_n$) calculé, après hydrolyse totale, de la façon suivante :

$$\overline{DP}_n = \frac{\% \, total \, de \, F}{\% \, total \, de \, G} + 1.$$

Il s'agit donc d'une moyenne de nombres (voir également "Production and use of inulin : Industrial reality with a promising future" (De Leenheer L., Carbohydrate as organic Raw Material Vol. III, pp. 67-92 (1996)) incorporé ci-après par référence).

Par "hydrolyse partielle", on entend une hydrolyse de la matière contenant des fructanes afin d'obtenir des oligosaccharides dont le DP maximal est de 20, de préférence de 10, et pus particulièrement de 9, et la teneur en F + G + GF est inférieure à 5%, et donc opposée à une hydrolyse totale qui impliquerait une dégradation complète en monomères.

Avantageusement, dans le procédé selon l'invention, les fructanes sont du type inuline ou du type lévane; l'inuline et le lévane étant caractérisés par la présence principale de liens fructosyl-fructose respectivement du type β-(2→1) et du type β-(2→6) (voir également le document cité dans le paragraphe précédent).

L'inuline peut être de l'inuline naturelle ou être produite par des micro-organismes. Dans l'inuline naturelle, le DP varie en général de 2 à 60, et dépend de l'origine végétale, de l'âge de la plante, de la durée et des conditions de sa conservation ainsi que du procédé d'extraction éventuel. L'inuline peut être extraite de la chicorée (Cichorium intybus), du dahlia (Dahlia variabilis), du topinambour (Helianthus tuberosus) ou du fond d'artichaut (Cynara scolymus). L'inuline peut aussi être extraite des plantes qui ont été génétiquement manipulées. Un procédé de transformation génétique de telles plantes est décrit notamment dans la demande de brevet WO94/14970. Le DP des fructanes obtenu de telles plantes dépasse aisément 10000.

Une inuline naturelle apte à être utilisée dans le procédé selon l'invention est par exemple une inuline ayant un DP moyen de 27 et exempte de G, F et GF. Cette inuline est commercialisée sous le nom SIGMA ® avec référence I-2255, I-3754 et I-2880 selon que l'inuline est extraite respectivement de la chicorée, du dahlia ou du topinambour.

Une autre inuline naturelle apte à être utilisée est une inuline extraite de la chicorée qui est commercialisée par ORAFTI sous le nom Raftiline ® HP. Ce produit a un DP moyen d'au moins 23 et contient au maximum, en totalité par rapport aux autres saccharides, 0,5% de G, F et GF (voir également la fiche de produit datée du 04/96 distribuée par ORAFTI). La production d'un tel produit est décrite dans la demande de brevet WO96/01849. Selon cette demande, on part d'une solution d'inuline extraite de la chicorée qui a une température de 65 °C. Cette solution est mise dans un état métastable et ensuite rapidement refroidie. Puis on ajoute des germes d'inuline et on obtient un précipité d'inuline fractionnée dans la solution. Ce précipité est séparé de la solution, lave et: séché. L'inuline fractionnée obtenue est exempte d'impuretés, a un DP moyen de préférence compris entre 20 et 40, et contient moins de 2% de F, G et GF.

Il est évident que dans le procédé selon l'invention, on peut utiliser le précipité lavé tel quel comme matière première.

Le DP d'une inuline produite par des microorganismes peut varier jusqu'à des valeurs de l'ordre de 60000. Une telle inuline est, par exemple, synthétisée à partir de saccharose par Aspergillus sydowi conidia en présence de L-cystéine, comme décrit dans l'article "Characteristics and Applications of a Polyfructan Synthesized from Sucrose by Aspergillus sydowi conidia" (T. Harada et al., Food Hydrocolloids, Vol. 7, No.1, pp. 23-28 (1993)). L'obtention d'une inuline "bactérienne" par une fructosyltransférase de Streptococcus mutans est décrite dans "Genetic and Antigenic Comparison of Streptococcus mutans Fructosyltransferase and Glucanbinding Protein" (J. Aduse-Opoku, FEMS Microbiology Letters 59, pp. 279-282 (1989)).

Le lévane se présente dans la nature surtout dans des graminées, mais l'extraction du lévane de ces plantes n'est pas exploitée actuellement industriellement. Le lévane est principalement obtenu par des micro-organismes, par exemple à partir de saccharose par l'activité de l'enzyme levansucrase de Bacillus subtilis tel que décrit dans "Modification of the Transfructosylation Activity of Bacillus subtilis levansucrase by Solvent Effect and Site-directed Mutagens" (R. Chambert et al., A. Fuchs (Ed.) Inulin and Inulincontaining crops, p. 259 (1993)). Il est évident que le lévane peut être également extrait (comme mentionné plus haut pour l'inuline) de plantes qui ont été génétiquement manipulées.

Selon l'invention, la matière contenant des fructanes est mise en solution dans l'eau préalablement au traitement hydrolytique. Il est souhaitable de préparer une solution de 5 à 25% par matière sèche, de préférence de 10 à 20% par matière sèche, de fructanes. Pourtant, en présence de fructanes ayant un DP élevé, il se peut que l'on ne puisse même pas dissoudre 5% de fructanes. En tout cas, il importe de veiller à ce que les fructanes soient complètement solubilisés.

Le traitement hydrolytique consiste en un traitement enzymatique partiel des fructanes. Ce traitement enzymatique partiel des fructanes est bien connu de l'Homme de l'Art.

Dans le cas où les fructanes sont de l'inuline, on utilise une préparation enzymatique ayant une activité endoinulinase. De telles préparations sont connues et peuvent être obtenues e.a. de cultures de Penicillium, Aspergillus, Fusarium ou Chrysosporium (voir également le document "The production of Fructooligosaccharides from Inulin or Sucrose Using Inulinase or Fructosyltransferase from Aspergillus ficuum" (Denpun Kagaku, Vol. 36, No. 2, pp. 103-111 (1989)), incorporé ici par référence).

Dans le cas où les fructanes sont du lévane, on utilise une préparation enzymatique ayant une activité endolevanase, comme décrit dans l'article "Metabolism in Microorganisms, Part II, Biosynthesis and Degradation of Fructans by Microbial Enzymes Cther than Levansucrase" (T. Uchiyama, Science and Technology of Fructans, p. 169 (1993)).

Il va de soi que les préparations enzymatiques ne peuvent avoir qu'une faible activité exo, de préférence, elles sont essentiellement exemptes d'activité exo. En général, le traitement enzymatique a lieu à une température de 58 à 62 °C et à un pH de 5,2 à 5,6, de préférence 5,4. La quantité d'unités d'enzymes (méthode NOVO) que l'on ajoute varie de 0,25 à 6 par gramme de matière sèche de fructanes dans la solution. De préférence, on utilise 0,4 à 1 unité d'enzyme par gramme. La réaction enzymatique prend alors respectivement 50 à 2 heures et 30 à 12 heures. Quand les fructanes ont un DP moyen élevé, par exemple supérieur à 50 comme notamment des fructanes produits par des micro-organismes, il est recommandé d'augmenter, de préférence de doubler, la quantité d'unités d'enzyme que l'on utilise et/ou d'augmenter la durée de réaction. La réaction enzymatique peut être arrêtée notamment en bouillant la solution hydrolysée et/ou en augmentant le pH à 8 - 9.

Il est évident que la solution polydispersée de saccharides que l'on obtient après le traitement enzymatique est purifiée (si nécessaire) par des traitements bien connus en soi. Eventuellement, on peut évaporer la solution afin d'obtenir un sirop d'une certaine matière sèche ou on peut sécher la solution, par exemple par un séchage par atomisation, afin d'obtenir une poudre de la granulométrie désirée.

La composition polydispersée de saccharides obtenue par le procédé selon l'invention pauvre en glucose (G), fructose (F) et saccharose (GF) comprend au moins 93,5%, de préférence 95%, en poids par MS, de fructo-oligosaccharides constitués de chaînes d'unités de fructose et de chaînes d'unités de fructose avec un glucose terminal, ces chaînes étant représentées respectivement par la formule $F_m$ et la formule $GF_n$, dans lesquelles n et m sont compris entre 2 et 20.

Une telle composition polydispersée de saccharides est décrite dans le brevet EP-B-0440074. Dans cette composition connue, le rapport entre les fructo-oligosaccharides non-réduisants ($GF_n$) et les fructo-oligosaccharides réduisants ($F_m$) n'est pas indiqué. Cependant, du procédé de préparation de cette composition, on peut déduire que la plupart des fructo-oligosaccharides sont des $GF_n$.

Une telle composition polydispersée de saccharides est également décrite dans l'article de A. De Bruyn et al. cité plus haut. Cette composition connue comprend principalement des fructo-oligosaccharides de la formule $GF_n$. Cette composition est commercialisée par ORAFTI sous le nom Raftilose ® L95 pour la forme liquide (sirop) et le nom Raftilose ® P95 pour la forme solide (poudre) (voir également les fiches de produit datées 05/95 distribuées par la société Raffinerie Tirlemontoise).

Ces compositions connues sont surtout utilisées dans l'industrie alimentaire. Par exemple, comme ingrédient alimentaire, elles se combinent de manière aisée aux autres ingrédients des produits alimentaires sans perturber en général les propriétés organoleptiques et visuelles des dits produits. En effet, ces compositions sont souvent utilisées en combinaison avec des polyalcools (étant des succédanés du sucre), afin d'améliorer certaines propriétés de ces polyalcools, notamment la coloration des produits. alimentaires que l'on fait cuire. Pourtant, ces compositions connues présentent l'inconvénient que dans certaines utilisations, telles que la pâtisserie, elles n'améliorent pas suffisamment la coloration.

En outre, la préparation de ces compositions connues est complexe et coûteuse.

La composition polydispersée de saccharides obtenue par le procédé selon l'invention évite les inconvénients des compositions connues, et présente dans des produits alimentaires des propriétés organoleptiques et visuelles comparables ou améliorées par rapport aux compositions connues.

A cet effet, les fructo-oligosaccharides comprennent plus de 43% en poids de fructo-oligosaccharides de formule $F_m$.

Avantageusement, les. fructo-oligosaccharides comprennent plus de 45%, de préférence plus de 50%, en poids de fructo-oligosaccharides de formule $F_m$.

De préférence, la composition obtenue par le procédé selon l'invention comprend moins de 5%, plutôt moins de 4%, en poids par MS totale de fructose, de glucose et de saccharose.

Avantageusement, la composition obtenue par le procédé de l'invention comprend tout au plus 1% en poids par MS de saccharose. Une telle composition convient encore mieux pour des diabètes.

De plus, la composition obtenue par le procédé selon l'invention comprend, de préférence, des fructo-oligosaccharides constitués de chaînes d'unités de fructose et de chaînes d'unités de fructose avec un glucose terminal, ces chaînes étant représentées respectivement par la formule $F_m$ et la formule $GF_n$, dans lesquelles n et m sont compris entre 2 et 10, plus particulièrement entre 2 et 9.

En outre, la composition polydispersée de saccharides obtenue par le procédé selon l'invention est essentiellement exempte d'inulo-oligosaccharides ayant un degré de polymérisation de plus de 10, de préférence de plus de 9, et, par conséquent, sa solution aqueuse à une concentration de 75%, et normalement même de 77%, en poids en matière sèche, reste limpide lors d'un stockage prolongé, même pendant plusieurs années, même à température ambiante.

Les liens entre les unités de fructose peuvent être du type $\beta$-$(2\rightarrow1)$ ou du type $\beta$-$(2\rightarrow6)$.

Avantageusement, la composition obtenue par le procédé selon l'invention est définie ci-dessus.

Les compositions obtenues par le procédé selon l'invention sont particulièrement adéquates pour être utilisées dans l'alimentation humaine ou animale comme agents de masse, édulcorants, aliments faiblement caloriques ou faiblement cariogènes, produits bifidogènes ou améliorant la flore intestinale, produits à effet de fibres alimentaires, agents diminuant le taux de cholestérol, ou encore pour améliorer la tolérance des produits alimentaires.

Ces compositions sont aussi particulièrement adéquates pour être utilisées dans des produits pharmaceutiques et/ou cosmétiques.

Les exemples suivants illustrent de manière non limitative l'objet de la présente invention.

### Exemple 1

La matière première contenant des fructanes est de l'inuline extraite de la chicorée ayant un DP moyen de 27 et exempte de F, G et GF. Il s'agit d'une inuline commercialisée sous le nom SIGMA ® . De cette inuline, on prépare une solution de 10% MS, on adapte le pH de la solution jusqu'à une valeur de 8 et on chauffe pendant 15 minutes à 90 °C afin d'obtenir une solution claire. On refroidit la solution claire jusqu'à 65 °C avant de la tamponner jusqu'à un pH de 5,4.

Ensuite, on ajoute 0,6 unités de l'enzyme A. Ficuum endo-inulinase (NOVO) par gramme de MS d'inuline pendant un traitement de 24 heures en maintenant la température à 60 °C. L'hydrolyse enzymatique est arrêtée en portant à ébullition la solution hydrolysée après avoir porté le pH à 8. La solution polydispersée de saccharides ainsi obtenue est ensuite décolorée et dessalée selon les procédés connus de l'Homme de l'Art. Le solvant de la solution polydispersée est ensuite évaporé pour obtenir un sirop de 75% MS aisément stockable.

On a déterminé le rapport entre les différents saccharides dans la solution obtenue par GS (Gas Chromatography) (tableau 1).

Ce rapport est déterminé pour une composition selon les procédés de l'état de la technique décrit plus haut et utilisés pour caractériser les produits Raftilose ® L95 commercialisés par ORAFTI.

Tableau 1

| | La composition obtenue par le procédé de l'invention | La composition de l'état de la technique Raftilose ® L95 |
|---|---|---|
| **POLYSACCHARIDE** | % carbohydrate par MS | % carbohydrate par MS |
| Fructose | 1,5 | 0,55 |
| Glucose | 0,1 | 0,04 |
| DFA * | 0,5 | 0,12 |
| Saccharose | 0 | 3,52 |
| $F_2$ | 1,58 | 0,41 |
| $GF_2$ | 0,18 | 4,61 |

(suite)

| | La composition obtenue par le procédé de l'invention | La composition de l'état de la technique Raftilose ® L95 |
|---|---|---|
| **POLYSACCHARIDE** | % carbohydrate par MS | % carbohydrate par MS |
| $F_3$ | 32 | 6,51 |
| $GF_3$ | 3,16 | 15,18 |
| $F_4$ | 31,11 | 13,42 |
| $GF_4$ | 5,98 | 21,14 |
| $F_5$ | 10,90 | 8,15 |
| $GF_5$ | 4,57 | 16,81 |
| $F_6$ | 6,50 | 8,56 |
| $GF_6$ | 1,05 | 2,31 |
| $F_7$ | 0,7 | 0,72 |
| $GF_7$ | 0,18 | 0,36 |
| $F_8$ | 0 | 0,21 |
| $GF_8$ | 0 | 0,33 |
| $F_9$ | 0 | 0 |
| DP > 10 | | 0,17 |
| **TOTAL** | 100 | 100 |
| (* : DFA = di-fructose anhydride) | | |

## Exemple 2

La matière première est de l'inuline commercialisée par ORAFTI sous le nom Raftiline ® HP. Cette inuline contient plus de 99,5% par MS de l'inuline d'un DP moyen d'au moins 23. On prépare une solution de 15% MS. On adapte le pH de la solution jusqu'à une valeur de 8,5 et on chauffe pendant 20 minutes à 90 °C afin d'obtenir une solution claire. Les autres étapes de ce procédé sont celles décrites dans l'exemple 1.

De la manière similaire à l'exemple 1, on détermine la distribution des principaux saccharides du produit obtenu comparée à un produit obtenu selon l'état de la technique, le Raftilose ® L95 commercialisé par Raffinerie Tirlemontoise (tableau 2).

Tableau 2

| | La composition de l'état de la technique Raftilose ® L95 | La composition obtenue par le procédé de l'invention |
|---|---|---|
| | % carbohydrate par MS | % carbohydrate par MS |
| Fructose | 0,5 | 2,5 |
| Saccharose | 3,6 | 0,3 |
| Glucose | 0,1 | 0,1 |
| $F_3$ | 6,4 | 31,7 |
| $F_m$ | 35 | 78,2 |
| $GF_n$ | 60,8 | 19 |
| FOS | 95,8 | 97,2 |
| $F_m$ (% p.r. FOS) | 37 | 81 |
| $GF_n$ (% p.r. FOS) | 63 | 19 |

On a également déterminé l'équivalent dextrose, la viscosité et l'hygroscopicité pour la composition obtenue par le procédé selon l'invention et la composition de l'état de la technique. L'équivalent dextrose est respectivement d'environ 10 et environ 24. La viscosité a été déterminée pour une solution respectivement de 77 et 50% MS à une température respective de 10 et 20 °C (tableau 3).

Tableau 3

| Concentration degrés BRIX | Température °C | La composition obtenue par le procédé de l'invention | La composition de l'état de la technique Raftilose ® L95 |
|---|---|---|---|
| | | Viscosité mPas | Viscosité mPas |
| 77 | 20 | 10000 | 16000 |
| 77 | 10 | 24700 | 57000 |
| 50 | 20 | 23,5 | 29 |
| 50 | 10 | 32 | 45 |

Afin de comparer la rétention d'eau de la composition obtenue par le procédé selon l'invention à celle de la composition selon l'état de la technique, on a déterminé les modifications de poids en fonction du pourcentage d'humidité relative à température ambiante. Les résultats sont donnés dans le tableau 4.

Tableau 4

| Humidité relative | Composition de l'état de la technique Raftilose ® L95 | Composition obtenue par le procédé de l'invention |
|---|---|---|
| °C | Différence de poids % | Différence de poids % |
| 23 | - 4,2 | - 3,4 |
| 44 | - 2,0 | - 0,2 |
| 66 | + 4,7 | + 6,0 |
| 80 | + 15,7 | + 18,2 |

La composition obtenue par le procédé selon l'invention est caractérisée par une modification de poids plus importante, et est donc plus hygroscopique que la composition selon l'état de la technique. Cela peut être un avantage dans certaines applications, notamment dans la préparation des cakes.

Ces deux exemples démontrent que le procédé selon l'invention est moins complexe que les procédés selon l'état de la technique. En outre le procédé selon l'invention est moins coûteux, ainsi que la composition obtenue par le procédé selon l'invention.

Il ressort des tableaux 1 et 2 que le rapport $F_m/GF_n$ de la composition obtenue par le procédé selon l'invention est différent de celui de la composition selon l'état de la technique et que la composition obtenue par le procédé selon l'invention peut être recommandée aux diabétiques. Les tableaux 3 et 4 démontrent que la composition obtenue par le procédé selon l'invention peut être également recommandée pour certaines applications.

## Exemple 3

La matière première est de l'inuline obtenue par l'action d'une fructosyltransférase de Streptococcus mutans, qui contient plus de 99,5% par MS de l'inuline d'un DP moyen d'environ 25000. On prépare une solution de 10% MS. On adapte le pH de la solution jusqu'à une valeur de 8 et on chauffe pendant 20 minutes à 90 °C afin d'obtenir une solution claire. On refroidit la solution claire jusqu'à 65 °C avant de la tamponner jusqu'à un pH de 5,4.

Ensuite, on ajoute 12 unités d'endo-inulase nécessaire par gramme de MS d'inuline, laquelle agit pendant 2 heures en maintenant la température à 60 °C. L'hydrolyse enzymatique est arrêtée en bouillant la solution hydrolysée après avoir augmenté le pH à 8,5 - 9. La solution polydispersée de saccharides ainsi obtenue est ensuite décolorée et dessalée de la manière habituelle.

On a déterminé le rapport entre les différents saccharides dans la solution obtenue par HPLC (High Pressure Liquid Chromatographie) (tableau 5).

Tableau 5

| | La composition obtenue par le procédé de l'invention |
|---|---|
| | **% carbohydrate par MS** |
| Fructose | 3,55 |
| Glucose | 0,11 |
| Saccharose | 0,11 |
| Other DP = 2 | 4 |
| $F_3$ | 30,85 |
| DP = 3 | 23,6 |
| DP = 4 | 11,67 |
| DP 5 | 26,11 |
| Total | 100 |

## Exemple 4

Cet exemple se rapporte à l'utilisation de la composition polydispersée de saccharides (CPS) obtenue par le procédé selon l'invention comparée à celle des produits selon l'état de la technique dans des applications diverses. On compare l'aspect, la structure, la coloration, la texture, la sensation en bouche et le goût des compositions alimentaires obtenues. Chaque application est présentée selon le schéma suivant :

1. Ingrédients et proportions
2. Recette : méthode de préparation
3. Résultats (comparaison des trois préparations)

Colonne 1 :   la référence, c'est-à-dire l'application préparée sans l'addition d'une composition polydispersée de saccharides

Colonne 2 :   l'application préparée avec l'addition d'une composition polydispersée de saccharides selon l'état de technique (composition commercialisée sous le nom Raftilose ® L95)

Colonne 3 :   l'application préparée avec l'addition d'une composition polydispersée de saccharides obtenue par le procédé selon la présente invention (composition décrite dans l'exemple 2)

4. Conclusions de la comparaison. Si pas de mention particulière, les autres critères donnent des résultats identiques

*Application 1 : lait chocolaté*

## 1. INGREDIENTS

Préparation de 300 g de chaque lait chocolaté

| Lait chocolaté | 1 | | 2 | | 3 | |
|---|---|---|---|---|---|---|
| | % | g | % | g | % | g |
| Sucre | 5,3 | 15,9 | - | - | - | - |
| Cacao en poudre (De Zaan : D-11-A) | 1,5 | 4,5 | 1,5 | 4,5 | 1,5 | 4,5 |
| Carraghénanes granulacta SGI-1 ® | 0,02 | 0,06 | 0,02 | 0,06 | 0,02 | 0,06 |
| Aspartame | - | - | 0,02 | 0,06 | 0,02 | 0,06 |
| CPS | - | - | 7 | 20,51 | 7 | 21,1 |
| Lait demi-écrémé | 92,4 | 277,2 | 90,7 | 272,5 | 90,7 | 272 |

(suite)

| Lait chocolaté | 1 | | 2 | | 3 | |
|---|---|---|---|---|---|---|
| | % | g | % | g | % | g |
| Total | 100 | 300 | 100 | 300 | 100 | 300 |

## 2. METHODE

- mélanger les produits secs et les disperser dans le lait
- mixer pendant 30 secondes
- chauffer 10 secondes à 75 °C
- refroidir jusqu'à température du frigo

## 3. RESULTATS

| Lait chocolaté | 1 | 2 | 3 |
|---|---|---|---|
| Goût | assez sucré chocolaté | assez sucré chocolaté | assez sucré chocolaté |
| Sensation en bouche | moins onctueux | plus onctueux | onctueux |
| Onctuosité | + | +++ | ++ |

## 4. CONCLUSIONS

On ne constate pas de différence de goût entre les trois préparations. La composition polydispersée de saccharides a un effet positif sur l'onctuosité du lait chocolaté (plus onctueux que la référence). Le lait chocolaté préparé avec la composition polydispersée de saccharides selon l'état de la technique est plus onctueuse que celle préparée avec la composition polydispersée obtenue par le procédé selon l'invention.

*Application 2 : pudding à la vanille.*

## 1. INGREDIENTS

Préparation de 500 g de chaque pudding.

| Pudding vanille | 1 | | 2 | | 3 | |
|---|---|---|---|---|---|---|
| | % | g | % | g | % | g |
| Poudre de lait écrémé | 10,1 | 50,5 | 10,1 | 50,5 | 10,1 | 50,5 |
| Sucre | 10 | 50 | - | - | - | - |
| Amidon de maïs (SF 6304 ® - Cerestar) | 1 | 5 | 1 | 5 | 1 | 5 |
| Stabilisant (Aubygel MR50 ® Sanofi) | 0,1 | 0,5 | 0,1 | 0,5 | 0,1 | 0,5 |
| β-carotène | 0,01 | 0,05 | 0,01 | 0,05 | 0,01 | 0,05 |
| Aspartame | - | - | 0,03 | 0,15 | 0,03 | 0,15 |
| Arôme vanille | 0,1 | 0,5 | 0,1 | 0,5 | 0,1 | 0,5 |
| CPS | - | - | 13,3 | 64,9 | 13,3 | 64,9 |
| Lait entier | 75,4 | 377 | 75,4 | 377 | 75,4 | 377 |
| Lait écrémé | 3,3 | 16,5 | - | - | - | - |

(suite)

| Pudding vanille | 1 | | 2 | | 3 | |
|---|---|---|---|---|---|---|
| | % | g | % | g | % | g |
| Total | 100 | 500 | 100 | 500 | 100 | 500 |

## 2. METHODE

- mélanger les produits secs sauf l'aspartame
- mélanger (mixer) les liquides dans le lait
- mélanger les produits secs et les liquides et mixer pendant 30 secondes
- chauffer pendant 30 minutes à 95 °C
- ajouter l'aspartame et bien mélanger
- verser dans différents petits pots
- refroidir, poser le couvercle et conserver à température du frigo

## 3. RESULTATS

| Pudding vanille | 1 | 2 | 3 |
|---|---|---|---|
| Texture (mesure de dureté en g) | plat, assez liquide (8) | plus ferme (11,5) | ferme (9,5) |
| Goût | plus sucré | moins sucré | moins sucré |
| Sensation en bouche | moins onctueux | onctueux | plus onctueux |
| Onctuosité | + | ++ | +++ |

## 4. CONCLUSIONS

Les puddings réalisés avec la composition polydispersée de saccharides ont une meilleure structure que la référence. Il sont plus fermes, plus solides. Le pudding réalisé selon l'état de la technique est plus ferme que celui contenant le produit obtenu par le procédé de l'invention.

Les trois puddings sont tous fort crémeux, mais on note une différence entre eux : ceux contenant une composition polydispersée de fructanes sont plus crémeux que la référence, le pudding contenant le produit obtenu par le procédé de l'invention est plus crémeux que celui réalisé avec une composition polydispersée selon l'état de la technique.

La différence au point de vue de la sensation en bouche est cependant très faible entre les deux dernières préparations.

*Application 3 : mousse au chocolat.*

## 1. INGREDIENTS

Préparation de 500 g de chaque mousse.

| Mousse au chocolat | 1 | | 2 | | 3 | |
|---|---|---|---|---|---|---|
| | % | g | % | g | % | g |
| Poudre de lait 7 écrémé | | 35 | 7 | 35 | 7 | 35 |
| Sucre | 17,5 | 87,5 | - | - | - | - |
| Cacao en poudre (De Zaan, D-11-A) | 4 | 20 | 4 | 20 | 4 | 205 |

(suite)

| Mousse au chocolat | 1 | | 2 | | 3 | |
|---|---|---|---|---|---|---|
| | % | g | % | g | % | g |
| Filgel (Quest 9323 ® ) | 2,1 | 10,5 | 2,1 | 10,5 | 2,1 | 10,5 |
| Gélatine (Sanofi 80 Bls ® ) | 0,5 | 2,5 | 0,5 | 2,5 | 0,5 | 2,5 |
| Aspartame | - | - | 0,05 | 0,25 | 0,05 | 0,25 |
| Crème (35% M. G.) | 6,3 | 31,5 | 6,3 | 31,5 | 6,3 | 31,5 |
| CPS | - | - | 23,3 | 113,7 | 23,3 | 117,1 |
| Lait écrémé | 62,6 | 313 | 56,8 | 286,7 | 56,8 | 283,4 |
| Total | 100 | 500 | 100 | 500 | 100 | 500 |

## 2. METHODE

- mélanger les produits secs sauf l'aspartame et mélanger (mixer) les liquides
- mélanger les produits secs et les liquides, mixer pendant 30 secondes et chauffer pendant 30 secondes à 90 °C
- ajouter l'aspartame et mixer pendant 30 secondes
- refroidir et placer une nuit au frigo
- battre 15 minutes à l'aide d'un batteur Hobart avec "fouet"

## 3. RESULTATS

| Mousse au chocolat | 1 | 2 | 3 |
|---|---|---|---|
| Poids avant | 93,5 | 89 | 89 |
| Poids après | 44 | 35,5 | 37 |
| Overrun | 113 | 150 | 140 |

| Mousse au chocolat | 1 | 2 | 3 |
|---|---|---|---|
| Texture | plutôt liquide | assez ferme | assez ferme |
| Aspect | assez foncé | clair | clair |
| Goût | sucré | sucré, légèrement amer | sucré, légèrement amer |
| Sensation en bouche | visqueux, lourd | très onctueux | très onctueux |

## 4. CONCLUSIONS

La mousse au chocolat de référence présente une structure très visqueuse, mais les mousses réalisées avec la composition polydispersée de saccharides ne présentent aucune différence entre elles, que ce soit en ce qui concerne la sensation en bouche, le goût, la structure ou l'aspect.

*Application 4 : bio-yoghourt*

## 1. INGREDIENTS

Préparation de 500 g de chaque bio-yoghourt.

| Bio-yoghourt | 1 | | 2 | | 3 | |
|---|---|---|---|---|---|---|
| | % | g | % | g | % | g |
| Lait entier | 94 | 470 | 90,5 | 452,9 | 90,5 | 452,4 |
| Lait écrémé en poudre | 1 | 5 | 1 | 5 | 1 | 5 |
| CPS | - | - | 3,5 | 17,1 | 3,5 | 17,6 |
| Ferment lactique | 5 | 25 | 5 | 25 | 5 | 25 |
| Total (g) | 100 | 500 | 100 | 500 | 100 | 500 |

## 2. METHODE

- ajouter le lait entier en poudre et la composition polydispersée au lait et mixer pendant 30 secondes
- chauffer pendant 8 minutes à 95 °C
- ajouter le ferment et bien mélanger
- mettre en pots et incuber à 40 °C jusqu'à pH 4,8
- refroidir rapidement et placer en chambre froide (24 heures)

## 3. RESULTATS

Les yoghourts ont été goûtés après 24 heures et 48 heures. Dans les deux cas, les résultats sont identiques.

| Bio-yoghourt | 1 | 2 | 3 |
|---|---|---|---|
| Structure | ± liquide | + ferme | + ferme |
| Texture | + aqueux | + onctueux | + onctueux |
| Goût | le même partout | | |

## 4. CONCLUSIONS

Les yoghourts réalisés avec la composition polydispersée de saccharides sont meilleurs que la référence, plus fermes et plus onctueux. On ne note pas de différences entre les yoghourts réalisés avec les deux compositions polydispersées de fructanes.

*Application 5 : sorbet à la fraise*

## 1. INGREDIENTS

Préparation de 1000 g de chaque sorbet.

| Sorbet fraise | 1 | 2 | 3 |
|---|---|---|---|
| Fraises | 485 | 485 | 485 |
| Sucre | 200 | - | - |
| CPS | - | 265 | 265 |
| Stabilisateur (Grindsted-Fructodan SL64 ®) | 5 | 5 | 5 |
| Aspartame | - | 0,8 | 0,8 |
| Eau | 310 | 245 | 245 |
| Total (g) | 1000 | 1000 | 1000 |

2. METHODE

- dégeler et écraser (mixer) les fraises, ajouter les autres ingrédients (sauf l'aspartame) et mixer pendant 20 minutes
- chauffer pendant 30 secondes à 90 °C, ajouter l'aspartame (vers 65-70 °C) et refroidir à 4 °C
- laisser reposer une nuit à 4 °C
- passer au Carpigiani (aération, congélation), conditionner en petits pots et placer au surgélateur pendant minimum 48 heures

3. RESULTATS

| Sorbet de fraises : overrun | 1 | 2 | 3 |
|---|---|---|---|
| Poids avant | 32 | 33 | 32,5 |
| Poids après | 20 | 20 | 19 |
| Overrun | 61 | 67 | 71 |

| Sorbet de fraises | 1 | 2 | 3 |
|---|---|---|---|
| Structure et goût | les mêmes partout | | |
| Sensation en bouche Onctuosité | + aqueux + | onctueux ++ | onctueux ++ |

4. CONCLUSIONS

Les sorbets contenant la composition polydispersée de saccharides sont plus onctueux que la référence.
Les sorbets réalisés avec les deux compositions polydispersées de fructanes donnent des résultats comparables entre eux.

*Application 6 : cake*

1. INGREDIENTS

| Cake | 1 % | 2 % | 3 % |
|---|---|---|---|
| Farine | 23,73 | 23,73 | 23,73 |
| Oeufs | 24 | 24 | 24 |
| Beurre | 20 | 20 | 20 |
| CPS | 0 | 16 | 16 |
| Lactitol | 24 | 12 | 12 |
| Acésulfam K | 0,05 | 0,05 | 0,05 |
| Levure chimique V90® | 0,2 | 0,2 | 0,2 |
| Levure chimique BPpyro® | 0,02 | 0,02 | 0,02 |
| Eau | 8 | 4 | 4 |
| Total | 100 | 100 | 100 |

## 2. METHODE

- laisser ramollir le beurre et ajouter les autres ingrédients
- mélanger les produits à l'aide d'un ustensile de cuisine pendant 3 minutes
- transvaser le tout dans un moule et enfourner à 210 °C

## 3. RESULTATS

Les trois cakes ont cuit ensemble durant 43 minutes. Les cakes contenant la composition polydispersée de saccharides sont meilleurs que la référence : ils ont une couleur brune. Par contre, le cake de référence a une couleur jaune pâle.

Il y a une différence de couleur entre les deux cakes contenant de la CPS. Le cake préparé avec la CPS obtenue par le procédé selon l'invention a une couleur plus brune comparée à celle du cake préparé avec la CPS selon l'état de la technique. Les couleurs brunes sont beaucoup plus souhaitées dans ce genre de produits.

*Application 7 : sablé*

## 1. INGREDIENTS

| Sablé | 1 % | 2 % | 3 % |
|---|---|---|---|
| Farine | 45,35 | 45,35 | 45,35 |
| Oeufs | 7,6 | 7,6 | 7,6 |
| Beurre | 24,2 | 24,2 | 24,2 |
| CPS | 0 | 10,1 | 10,1 |
| Lactitol | 15 | 7,5 | 7,5 |
| Sucre vanillé | 0,8 | 0,8 | 0,8 |
| Acésulfam K | 0,05 | 0,05 | 0,05 |
| Levure | 0,6 | 0,6 | 0,6 |
| Sel | 0,3 | 0,3 | 0,3 |
| Eau | 6,1 | 3,5 | 3,5 |
| Total | 100 | 100 | 100 |

## 2. METHODE

- laisser ramollir le beurre et y ajouter les ingrédients
- mélanger à l'aide d'un ustensile de cuisine pour l'homogénéisation et transvaser dans des moules
- cuire au four à 178 °C

## 3. RESULTATS

Les trois sablés ont été cuits ensemble pendant 14 minutes. Le sablé préparé sans la composition polydispersée de saccharides a une couleur très pâle. Les deux autres sablés sont agréablement colorés, le sablé contenant la CPS selon l'état de la technique a un effet moins prononcé.

**Revendications**

1. Procédé de préparation d'une composition polydispersée de saccharides dans lequel un matériau contenant des fructanes avec un degré de polymérisation moyen égal ou supérieur à 7 et contenant tout au plus 3,5% en poids par matière sèche au total de glucose, de fructose et de saccharose et qui est complètement solubilisé dans l'eau, est soumis à une hydrolyse partielle avec un enzyme ayant une activité endo et étant essentiellement dépourvue d'activité exo, conduisant directement, sans nécessiter une séparation chromatographique, à une composition polydispersée de saccharides comprenant au moins 93,5% en poids par matière sèche de fructo-oligosaccharides constitués de chaînes d'unités de fructose de la formule $F_m$ et de chaînes d'unités de fructose avec un glucose terminal de la formule $GF_n$, n et m étant compris entre 2 et 10, comprenant plus de 43% en poids par matière sèche de fructo-oligosaccharides de la formule $F_m$, et comprenant une teneur en glucose, fructose et saccharose au total inférieure à 5% en poids par matière sèche, et dont la solution aqueuse à une concentration de 75% en poids en matière sèche reste limpide lors d'un stockage, à température ambiante.

2. Procédé de préparation selon la revendication 1, **caractérisé en ce que** les fructo-oligosaccharides obtenus correspondent à la formule $F_m$ ou $GF_n$, dans laquelle n et m sont compris entre 2 et 9.

3. Procédé de préparation selon la revendication 1 ou 2, **caractérisé en ce que** la composition polydispersée de saccharides obtenue reste limpide en solution aqueuse à une concentration de 77%, en poids en matière sèche, à température ambiante.

4. Procédé de préparation selon l'une quelconque des revendications précédentes, **caractérisé en ce que** la composition polydispersée de saccharides comprend au moins 95% en poids par matière sèche de fructo-oligosaccharides comprenant plus de 45% en poids par matière sèche de fructo-oligosaccharides de la formule $F_m$, et que ladite matière contient tout au plus 2% en poids par matière sèche au total de glucose, fructose et saccharose.

5. Procédé de préparation selon l'une quelconque des revendications précédentes, **caractérisé en ce que** les fructanes sont du type inuline et que l'hydrolyse partielle est obtenue à l'aide d'une préparation enzymatique ayant une activité endo-inulase, essentiellement exempte d'activité exo.

6. Procédé de préparation selon l'une quelconque des revendications 1 à 4, **caractérisé en ce que** les fructanes sont du type lévane et que l'hydrolyse partielle est obtenue à l'aide d'une préparation enzymatique ayant une activité endo-lévanase, essentiellement exempte d'activité exo.

7. Procédé de préparation selon l'une quelconque des revendications précédentes, **caractérisé en ce que** l'hydrolyse est effectuée en solution aqueuse à une température de 58 à 62 °C, à un pH de 5.2 à 5.6 et à l'aide d'une préparation d'enzyme contenant de 0,25 à 12, de préférence de 0,25 à 6, unités d'enzymes (méthode NOVO) par gramme de matière sèche de fructanes.

8. Procédé de préparation selon l'une quelconque des revendications précédentes, selon lequel la composition dispersée de saccharides obtenue après l'hydrolyse partielle est ensuite décolorée et dessalée.

**Claims**

1. Method for preparing a polydispersed saccharide composition, wherein a fructan-containing material with an average degree of polymerization of greater than or equal to 7, containing at most 3.5% by weight relative to the dry matter in total of glucose, fructose and saccharose and which is completely dissolved in water is subjected to a partial hydrolysis with an enzyme having an endo-activity and being essentially free of exo-activity, which gives directly, without using a chromatography separation, a polydispersed saccharide composition comprising at least 93.5% by weight relative to the dry matter of fructo-oligosaccharides consisting of chains of fructose units of formula $F_m$ and of chains of fructose units with a terminal glucose of formula $GF_n$, n and m being between 2 and 10 and comprising more than 43% by weight relative to the dry matter of fructo-oligosaccharides of formula $F_m$ and comprising a glucose, fructose and saccharose content in total of less than 5% by weight relative to the dry matter, the aqueous solution of which at a concentration of 75% by weight relative to the dry matter remaining clear during storage at room temperature.

2. Method of preparation according to claim 1, **characterized in that** the fructo-oligosaccharides obtained correspond

to the formula $F_m$ or $GF_n$, in which n and m are between 2 and 9.

3. Method of preparation according to claim 1 or 2, **characterized in that** the polydispersed saccharide composition obtained remains clear in aqueous solution at a concentration of 77%, by weight relative to the dry matter, at room temperature.

4. Method of preparation according to any one of the preceding claims, **characterized in that** the polydispersed saccharide composition comprises at least 95% by weight relative to the dry matter of fructo-oligosaccharides comprising more than 45% by weight relative to the dry matter of fructo-oligosaccharides of formula $F_m$ and **in that** the said material contains at most 2% by weight relative to the dry matter in total of glucose, fructose and saccharose.

5. Method of preparation according to any one of the preceding claims, **characterized in that** the fructans are of the inulin type and **in that** the partial hydrolysis is obtained using an enzymatic preparation having an endoinulase activity, essentially free of exo-activity.

6. Method of preparation according to any one of claims 1 to 4, **characterized in that** the fructans are of the levan type and **in that** the partial hydrolysis is obtained using an enzymatic preparation having an endolevanase activity, essentially free of exo-activity.

7. Method of preparation according to any one of the preceding claims, **characterized in that** the hydrolysis is carried out in aqueous solution at a temperature of 58 to 62 °C, at a pH of 5.2 to 5.6 and using an enzyme preparation containing 0.25 to 12, preferably 0.25 to 6, enzyme units (NOVO method) per gram of dry matter of fructans.

8. Method according to any one of the preceding claims, wherein the dispersed saccharide composition obtained after partial hydrolysis is thereafter decolorized and desalted.

**Patentansprüche**

1. Verfahren zur Herstellung einer polydispersen Zusammensetzung von Sacchariden, bei welchem ein Material, das Fructane mit einem mittleren Polymerisationsgrad gleich oder höher als 7 enthält und das einen totalen Gehalt von höchstens 3,5 Gew.-% der Trockensubstanz an Glucose, Fructose und Saccharose enthält und das vollständig im Wasser solubilisiert ist, einer partiellen Hydrolyse unterzogen wird mit einem Enzym, das eine Endo-Aktivität besitzt und das im Wesentlichen frei von einer Exo-Aktivität ist, welche unmittelbar, ohne dass eine chromatographische Trennung notwendig wäre, zu einer polydispersen Zusammensetzung von Sacchariden führt, welche mindestens 93,5 Gew.-% der Trockensubstanz an Fructo-Oligosacchariden enthält, die zusammengesetzt sind aus Ketten von Fructoseeinheiten mit der Formel $F_m$ und aus Ketten von Fructoseeinheiten mit einem endständigen Glucose der Formel $GF_n$, wobei n und m zwischen 2 und 10 liegen, und welche mehr als 43 Gew.-% der Trockensubstanz an Fructo-Oligosacchariden der Formel $F_m$ enthält, und einen Gehalt an Glucose, Fructose und Saccharose aufweist, der insgesamt geringer als 5% Gew.-% der Trockensubstanz ausmacht, und dessen wässerige Lösung mit einer Konzentration von 75 Gew.-% an Trockensubstanz während einer Aufbewahrung unter Raumtemperatur durchsichtig bleibt.

2. Herstellungsverfahren gemäß Anspruch 1, **dadurch gekennzeichnet, dass** die erzielten Fructo-Oligosaccharide der Formel $F_m$ oder $GF_n$ entsprechen, in welcher n und m zwischen 2 und 9 liegen.

3. Herstellungsverfahren gemäß Anspruch 1 oder 2, **dadurch gekennzeichnet, dass** die erzielte polydisperse Zusammensetzung der Saccharide in einer wässerigen Lösung bei einer Konzentration von 77 Gew.-% an Trockensubstanz bei Raumtemperatur durchsichtig bleibt.

4. Herstellungsverfahren gemäß irgendeinem der vorhergehenden Ansprüche, **dadurch gekennzeichnet, dass** die polydisperse Zusammensetzung der Saccharide mindestens 95 Gew.-% der Trockensubstanz an Fructo-Oligosacchariden enthält, welche mehr als 45 Gew.-% der Trockensubstanz an Fructo-Oligosacchariden mit der Formel $F_m$ enthält, und dass die Trockensubstanz höchstens einen totalen Gehalt von 2 Gew.-% der Trockensubstanz an Glucose, Fructose und Saccharose enthält.

5. Herstellungsverfahren gemäß irgendeinem der vorhergehenden Ansprüche, **dadurch gekennzeichnet, dass** die Fructane von dem Typus des Inulins sind und dass die partielle Hydrolyse mithilfe einer enzymatischen Zubereitung erzielt wird, welche eine Endo-Inulase-Aktivität besitzt und im Wesentlichen frei von Exo-Aktivität ist.

6. Herstellungsverfahren gemäß irgendeinem der Ansprüche 1 bis 4, **dadurch gekennzeichnet, dass** die Fructane von dem Typus des Levans sind und dass die partielle Hydrolyse mithilfe einer enzymatischen Zubereitung erzielt wird, weiche eine EndoLevanase-Aktivität besitzt und im Wesentlichen frei von Exo-Aktivität ist.

7. Herstellungsverfahren gemäß irgendeinem der vorhergehenden Ansprüche, **dadurch gekennzeichnet, dass** die Hydrolyse in einer wässerigen Lösung durchgeführt wird, bei einer Temperatur von 58 bis 62°C, bei einem pH von 5,2 bis 5,6 und mithilfe einer enzymatischen Zubereitung, die zwischen 0,25 und 12, vorzugsweise zwischen 0,25 und 6 Einheiten der Enzyme (Methode NOVO) pro Gramm Trockensubstanz an Fructanen enthält.

8. Herstellungsverfahren gemäß irgendeinem der vorhergehenden Ansprüche, **dadurch gekennzeichnet, dass** die dispersierte Zusammensetzung der Saccharide, die nach der partiellen Hydrolyse erzielt wird, alsdann entfärbt und entsalzt wird.

**EP 0 917 588 B2**

RÉFÉRENCES CITÉES DANS LA DESCRIPTION

**Documents brevets cités dans la description**

- JP 5021074 A
- JP 6014792 A
- WO 9414970 A
- WO 9601849 A